(19) **Europäisches Patentamt**
**European Patent Office**
**Office européen des brevets**

(11) **EP 1 827 510 B1**

(12) **EUROPÄISCHE PATENTSCHRIFT**

(45) Veröffentlichungstag und Bekanntmachung des Hinweises auf die Patenterteilung:
**06.10.2010 Patentblatt 2010/40**

(21) Anmeldenummer: **05850146.1**

(22) Anmeldetag: **13.12.2005**

(51) Int Cl.:
*A61L 2/04* *(2006.01)*     *A61L 2/20* *(2006.01)*
*A61L 101/22* *(2006.01)*     *A61L 101/38* *(2006.01)*

(86) Internationale Anmeldenummer:
**PCT/DE2005/002240**

(87) Internationale Veröffentlichungsnummer:
**WO 2006/063569 (22.06.2006 Gazette 2006/25)**

(54) **VERFAHREN UND VORRICHTUNG ZUM DEKONTAMINIEREN VON GEGENSTÄNDEN, INSBESONDERE ZUM ENTGIFTEN ODER ENTSEUCHEN VON TEMPERATUREMPFINDLICHEM MILITÄRISCHEN KLEINGERÄT**

METHOD AND DEVICE FOR DECONTAMINATING ARTICLES, ESPECIALLY FOR DETOXICATING OR DISINFECTING TEMPERATURE-SENSITIVE SMALL MILITARY DEVICES

PROCEDE ET DISPOSITIF POUR DECONTAMINER DES OBJETS, EN PARTICULIER POUR DETOXIQUER OU DESINFECTER DES PETITS APPAREILS MILITAIRES SENSIBLES A LA TEMPERATURE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **13.12.2004 DE 102004062368**

(43) Veröffentlichungstag der Anmeldung:
**05.09.2007 Patentblatt 2007/36**

(73) Patentinhaber: **Kärcher Futuretech GmbH
71364 Winnenden (DE)**

(72) Erfinder:
• **TOEPFER, Hans-Joachim
71522 Backnang (DE)**

• **KOSTRON, Markus
71686 Remseck (DE)**

(74) Vertreter: **Duhme, Torsten et al
Witte, Weller & Partner
Patentanwälte
Postfach 10 54 62
70047 Stuttgart (DE)**

(56) Entgegenhaltungen:
**EP-A- 0 109 352     EP-A1- 0 109 352
WO-A-03/035118     DE-A1- 3 842 285
DE-A1- 19 823 163     GB-A- 2 055 289
US-A- 5 122 344     US-A- 5 122 344**

EP 1 827 510 B1

**Beschreibung**

Die vorliegende Erfindung betrifft eine Vorrichtung zum Dekontaminieren von Gegenständen, insbesondere zum Entgiften oder Entseuchen von temperaturempfindlichem, militärischen Kleingerät, nach Anspruch 1.

Die Erfindung betrifft ferner ein Verfahren zum Dekontaminieren von Gegenständen, insbesondere zum Entgiften oder Entseuchen von temperaturempfindlichem, militärischen Kleingerät, nach Anspruch 10.

Eine solche Vorrichtung und ein solches Verfahren sind aus US 5,122,344 bekannt.

Dekontaminieren im Sinne der vorliegenden Erfindung bedeutet das gezielte Beseitigen/Entfernen von radioaktiven Substanzen und/oder das gezielte Unschädlichmachen oder Entfernen von biologischen oder chemischen Substanzen, die ohne derartige Maßnahmen eine Gefahr für die Gesundheit oder sogar das Leben von Personen darstellen. Derartige Substanzen können die Folge von militärischen oder terroristischen Anwendungen entsprechender Kampfstoffe sein. Darüber hinaus können die Substanzen auch auf Grund von Unfällen zu einer Kontamination von Personen oder Gegenständen führen. Maßnahmen zur Dekontamination beinhalten das Entstrahlen (A-Dekontamination), das Entseuchen (B-Dekontamination) und das Entgiften (C-Dekontamination), wobei abhängig von der Art der Dekontamination unterschiedliche Dekontaminationsmittel und Vorgehensweisen zum Einsatz kommen. Die vorliegende Erfindung betrifft vor allem das Entgiften und Entseuchen von temperaturempfindlichem Kleingerät, beispielsweise von Zielfernrohren oder anderen optischen oder elektronischen Komponenten.

Aus DE 34 29 346 A1 ist ein Dekontaminationsverfahren bekannt, bei dem als Dekontaminationsmittel ein Gemisch aus Heißluft und Wasserdampf verwendet wird. An die Stelle der Heißluft soll auch jedes andere erwärmte Gas treten können. Vorgeschlagen wird insbesondere die Verwendung von Abgasen aus Verbrennungsprozessen. Maßgeblich für den Erfolg dieses bekannten Verfahrens sind im Wesentlichen die hohen Temperaturen von z.B. 170°C. Daher ist dieses bekannte Verfahren nicht dazu geeignet, temperaturempfindliche Gegenstände zu dekontaminieren. Darüber hinaus gewährleistet das bekannte Verfahren keine zuverlässige Entseuchung bei kritischen biologischen Kampfstoffen, wie z.B. Mykotoxinen oder Anthraxsporen.

Das aus DE 34 29 346 A1 bekannte Verfahren unterscheidet sich durch die Verwendung eines Heißluft/Heißgas-Dampf-Gemisches bei Umgebungsdruck von einem anderen bekannten Verfahren, das in der Beschreibungseinleitung der DE 34 29 346 A1 erwähnt ist.

Bei Letzterem erfolgt die Dekontamination allein durch heißen Dampf unter einem Druck von maximal 4 bar. In einer sich daran anschließenden Phase werden die dekontaminierten Gegenstände mit Heißluft getrocknet. Auch dieses bekannte Verfahren gewährleistet jedoch keine hinreichende Entseuchung von kritischen biologischen Kampfstoffen und es ist darüber hinaus nicht zur Entgiftung von temperaturempfindlichen Gegenständen geeignet.

Aus DE 36 25 847 A1 ist eine Dekontaminationskammer bekannt, die vor allem zur Dekontamination von Bekleidungsgegenständen vorgesehen ist. Auch in diesem Fall wird ein Heißgas-Dampfgemisch unter Verwendung von Rauch- bzw. Abgasen aus Verbrennungsvorgängen als Dekontaminationsmittel verwendet. Die Dekontamination erfolgt auch bei dieser bekannten Dekontaminationskammer mit hohen Temperaturen bei Umgebungsdruck.

Aus DE 34 13 743 A1 und aus DE 38 35 857 A1 sind jeweils Vorrichtungen bekannt, um Heißdampf zur Dekontamination von Bekleidungs- und Ausrüstungsgegenständen zu erzeugen. Die Verwendung von Heißdampf und ergänzend dazu die Verwendung von Verbrennungsabgasen als Dekontaminationsmittel macht jedoch auch diese bekannten Vorrichtungen ungeeignet für die Dekontamination von temperaturempfindlichen Ausrüstungsgegenständen. Darüber hinaus gewährleisten auch diese bekannten Verfahren keine hinreichende Dekontamination von kritischen biologischen Kampfstoffen.

Aus der sog. RKI-Liste, die vom Robert-Koch-Institut in Deutschland herausgegeben wird und die im Bundesgesundheitsblatt der Bundesrepublik Deutschland Nr. 1-2003 veröffentlicht ist, sind verschiedene Verfahren zur Desinfektion von Gegenständen bekannt. Unterschieden werden hier thermische Verfahren und chemische Verfahren. Erstere setzen in aller Regel Heißdampf zur Desinfektion ein, wobei die zu desinfizierenden Gegenstände in einer Kammer untergebracht sind, die wiederholt evakuiert wird und in die dann Dampfstöße hineingegeben werden. Dabei sind Verfahren erwähnt, die mit einer Dampftemperatur von 75°C arbeiten, was im Vergleich zu den vorbeschriebenen Dekontaminationsverfahren eine relativ niedrige Temperatur ist. Die chemischen Desinfektionsverfahren beruhen in erster Linie auf der Anwendung von chemischen Desinfektionsmitteln, wie beispielsweise Formaldehyd, Peressigsäure oder Chlor. Des Weiteren sind in der RKI-Liste chemothermische Desinfektionswaschverfahren erwähnt, bei denen eine Wäsche mit chemischen Desinfektions- und Reinigungsmitteln bei Temperaturen von 60°C bis 70°C erfolgt.

DE 29 21 915 A1 beschreibt eine Vorrichtung und ein Verfahren zum Sterilisieren von thermolabilen Materialien im medizinischen Bereich. Ziel ist es, die Sterilisation bei Gegenständen mit engen Rohren oder Schläuchen, wie etwa bei Endoskopen und Kathetern, befriedigend zu gestalten. Da kleine Kondensattröpfchen die Poren und Kapillare bei derartigen Gegenständen verschließen können und anschließend ein dampfförmiges Desinfektionsmittel absorbieren können, wird die Sterilisationskammer zunächst mehrfach abwechselnd mit einem Wirkstoffgas gespült und wieder entlüftet. Beispielsweise wird die Sterilisationskammer zunächst bei einem Unterdruck von beispielsweise 6 kPa mit Wasseraldehyddampf gefüllt. Das Einfüllen erfolgt bis zu einem Druck, der unterhalb des Sättigungsdrucks liegt, beispielsweise bis 16 kPa. Anschließend wird das Luft-Aldehyddampfgemisch wieder bis auch einen Wert von etwa 6 kPa abgesaugt. Dieser Vorgang wird mehrfach wiederholt, bis man davon ausgeht, dass auch kleinste Hohlräume des zu sterilisierenden Gegenstandes mit dem Wirkstoffgas gefüllt sind. Danach wird der Betriebsdruck in der Sterilisationskammer über den Sättigungswert des Wirkstoffs angehoben und während einer Einwirkzeit konstant gehalten. Die eigentliche Sterilisation erfolgt also unter einem Druck, der oberhalb des Sättigungsdruckkes liegt, so dass der Wirkstoff teilweise in kondensierter Form vorliegt.

Aus DE 698 21 825 T2 sind eine andere Vorrichtung und ein Verfahren zum Sterilisieren bekannt. Hier wird zunächst Luft aus einer Behandlungskammer abgezogen, um Vakuumbedingungen zu erreichen. Anschließend wird Wasserstoffperoxidgas in die Behandlungskammer zugeführt, wobei eine vorgegebene Menge zugeführt werden soll, damit das Wasserstoffperoxidgas in der Gasphase verbleibt. Die Konzentration an Wasserstoffperoxidgas wird mit Hilfe eines Konzentrationsmessgerätes bestimmt, das mit der Behandlungskammer verbunden ist.

EP 0 109 352 offenbart ein Verfahren und eine Vorrichtung zum Sterilisieren von Utensilien aus thermolabilen Materialien. Die zu sterilisierenden Gegenstände werden in eine luftdicht abschließbare Kammer gebracht. Nach Evakuierung bis auf ca. 3000 Pa wird Peressigsäure-Lösung von 4 % zur Verdampfung gebracht.

Auch bei dem Verfahren und der Vorrichtung aus der eingangs genannten US 5,122,344 wird ein Sterilisationsmittel in flüssiger Form in die Behandlungskammer eingebracht.

DE 38 42 285 A1 offenbart ein weiteres Verfahren und eine weitere Vorrichtung zum Sterilisieren oder Desinfizieren von Gegenständen. Bei dieser Vorrichtung kann ein Verdampfer außerhalb des Innenraums der Behandlungskammer angeordnet sein. Optional kann der Innenraum mit einer Vakuumpumpe verbunden sein. Eine Dosierung des Sterilisationsmittels erfolgt unmittelbar in der Vorrichtung.

Vor diesem Hintergrund ist es eine Aufgabe der vorliegenden Erfindung, ein Verfahren und eine Vorrichtung der eingangs genannten Art anzugeben, mit denen sich temperatur- und wasserempfindliche Ausrüstungsgegenstände, wie insbesondere optische oder elektronische Geräte, zuverlässig entseuchen lassen. Zudem sollen die neue Vorrichtung und das neue Verfahren einfach und robust sein, um eine Verwendung unter militärischen Einsatzbedingungen zu ermöglichen.

Diese Aufgabe wird gemäß einem Aspekt der Erfindung durch eine Vorrichtung der eingangs genannten Art gelöst, bei der die Vakuumpumpe dazu ausgebildet ist, den Innenraum auf einen Innendruck von weniger als 10 Pa, bevorzugt auf einen Innendruck von etwa 1 Pa zu evakuieren, bei der die Heizung ferner zumindest einen Wärmestrahler beinhaltet, der in dem Innenraum angeordnet ist, und bei der die Zuleitung vollständig beheizbar ist, um ein Kondensieren eines verdampften gasförmigen Dekontaminationsmittels beim Einleiten in den Innenraum zu unterbinden.

Die Aufgabe wird gemäß einem zweiten Aspekt der Erfindung durch ein Verfahren der eingangs genannten Art gelöst, bei dem der Innenraum auf einen Innendruck von weniger als 100 Pa, vorzugsweise auf einen Innendruck von weniger als 10 Pa, und noch weiter bevorzugt auf einen Innendruck von etwa 1 Pa evakuiert wird, wobei der Innenraum ferner mit zumindest einem Wärmestrahler beheizt wird, der in dem Innenraum angeordnet ist, wobei ein Druckanstieg in dem Innenraum beim Befüllen des Innenraums mit einem gasförmigen chemischen Dekontaminationsmittel bestimmt wird, und wobei das gasförmige chemische Dekontaminationsmittel in Abhängigkeit von dem Druckanstieg zugeführt wird.

Besonders vorteilhafte weiterbildungen der Erfindung sind den abhängigen Ansprüchen zu entnehmen.

Die vorliegende Erfindung beruht auf der Idee, temperaturempfindliche Gegenstände bei Unterdruck, im Idealfall im Vakuum, zu dekontaminieren. Dadurch lässt sich in der Kammer eine genau definierte Atmosphäre erzeugen, die abhängig von den verwendeten Dekontaminationsmitteln und abhängig von der eingestellten Temperatur eine zuverlässige Dekontamination der in der Kammer befindlichen Gegenstände gewährleistet. Die Durchführung der Dekontamination bei geringen Drücken und insbesondere im Vakuum ermöglicht die Reduzierung der Dekontaminationstemperaturen, ohne den Erfolg der Dekontamination zu beeinträchtigen. Daher lassen sich mit Hilfe des neuen Verfahrens und der neuen Vorrichtung auch temperaturempfindliche Gegenstände erfolgreich dekontaminieren, und zwar vor allem auch solche, die nicht mit reaktiven Chemikalien im nasschemischen Verfahren dekontaminiert werden können.

Gemäß der Erfindung beinhaltet die Heizung zumindest einen Wärmestrahler, insbesondere Infrarotstrahler, der oder die in dem Innenraum angeordnet sind, sowie eine Mantelheizung, die dazu ausgebildet ist, Innenwände der Vakuumkammer zu beheizen. Dementsprechend erfolgt die Beheizung bei dem erfindungsgemäßen Verfahren zumindest unter Zuhilfenahme von solchen Wärmestrahlern und unter Zuhilfenahme von einer solchen Mantelheizung. Bevorzugt ist die Mantelheizung als elektrische Mantelheizung ausgebildet.

Die Wärmestrahler besitzen den Vorteil, dass die zu dekontaminierenden Gegenstände auch im Vakuum sehr zuverlässig auf eine gewünschte Dekontaminationstemperatur gebracht werden können. Dies ist von besonderer Bedeutung, da die Gegenstände beim Evakuieren der Kammer abkühlen. Andererseits hat sich gezeigt, dass eine Mantelheizung die Gegenstände in der Kammer nur sehr schwer oder gar nicht aufheizen kann, da im fast vollständigen Vakuum ein Wärmeübertragungsmedium fehlt. Mit anderen Worten trägt die Verwendung von Wärmestrahlern dazu bei, die für eine erfolgreiche Dekontamination benötigten Temperaturen an den Gegenständen zu gewährleisten. Die Mantelheizung besitzt den Vorteil, dass eine Kondensation von Dekontaminationsmittel an den Innenwänden der Kammer verhindert wird. Dies sorgt dafür, das dem Dekontaminationsprozess kein Dekontaminationsmittel "entzogen" wird. Erst die Kombination beider Heizverfahren führt dazu, dass eine Dekontamination unter allen Einsatzbedingungen zuverlässig möglich ist.

Durch die vollständig beheizbare Zuleitung wird das Kondensieren eines verdampften Dekontaminationsmittels beim Einleiten in die Kammer noch zuverlässiger unterbunden. Die Prozessparameter beim Dekontaminieren können damit noch zuverlässiger gewährleistet werden. Infolgedessen ist auch der Erfolg der Dekontamination mit einer noch höheren Zuverlässigkeit gegeben.

Mit Hilfe des Druckmessers lässt sich die Konzentration des Dekontaminationsmittels in der Kammer sehr einfach und zuverlässig bestimmen, ohne dass dafür spezielle Gassensoren in der Kammer bereitgestellt werden müssen. Die Einstellung und/oder Überwachung der Dekontaminationsmittelkonzentration über den Innendruck in der Kammer ist darüber hinaus auch deshalb von großem Vorteil, weil bei den niedrigen Betriebsdrücken, die nach der vorliegenden Erfindung angewendet werden, nur relative wenige Gasmoleküle in der Kammer vorhanden sind, so dass eine Konzentrationsmessung mit Hilfe eines Gassensors in hohem Maße von statistischen Fehlern überlagert ist. Damit ermöglicht die bevorzugte Ausgestaltung eine besonders einfache, kostengünstige und zuverlässige Entseuchung.

In einer Ausgestaltung sind der zumindest eine Wärmestrahler und die Mantelheizung unabhängig voneinander steuerbar.

Diese Ausgestaltung besitzt den Vorteil, dass die Heizleistung im Verlauf des Dekontaminationsprozesses optimal gesteuert werden kann.

In einer weiteren Ausgestaltung ist die Mantelheizung dazu ausgebildet, sämtliche Innenwände der Vakuumkammer zu beheizen.

Mit anderen Worten erfolgt in dieser Ausgestaltung eine allseitige Beheizung der Vakuumkammer einschließlich von Türbereichen. Damit lässt sich ein "Wirkstoffverlust" auf Grund von Kondensatbildung optimal verhindern.

In einer weiteren Ausgestaltung ist die Heizung so ausgebildet, dass sie den Innenraum auf bis zu 200°C aufheizen kann.

Bei der Dekontamination von temperaturempfindlichen Gegenständen wird die Heizleistung so begrenzt, dass der Innenraum bzw. die darin befindlichen Gegenstände nicht wärmer als etwa 75°C werden. Untersuchungen der Anmelderin haben gezeigt, dass dieser Temperaturbereich einerseits hinreichend niedrig ist, um optische und/oder elektronische Komponenten und Geräte, wie sie vor allem bei militärischen Einsätzen benötigt werden, bei der Dekontamination nicht zu beschädigen. Andererseits gewährleistet dieser Temperaturbereich in Kombination mit den niedrigen Drücken, die mit der Vakuumkammer einstellbar sind, eine zuverlässige Entgiftung und Entseuchung. Andererseits erweitert eine Heizung nach dieser bevorzugten Ausgestaltung den Anwendungsbereich der Vorrichtung, was für militärische Einsätze von großem Vorteil ist.

In einer weiteren Ausgestaltung beinhalten die neue Vorrichtung und das neue Verfahren zumindest einen UV-Strahler, der in dem Innenraum zum Bestrahlen der Gegenstände angeordnet ist.

Die ergänzende Bereitstellung von zumindest einem UV-Strahler erweitert das Anwendungsspektrum der neuen Vorrichtung vor allem bei der Entseuchung. Die Flexibilität und Einsatzbandbreite wird daher weiter erhöht.

In einer weiteren Ausgestaltung beinhaltet die zweite Anordnung einen Verdampfer zum kondensatfreien Verdampfen eines in dem Vorratsbehälter bereitgestellten Dekontaminationsmittels.

Diese Ausgestaltung vergrößert den Anwendungsbereich der neuen Vorrichtung und des neuen Verfahrens noch weiter. Insbesondere wird hierdurch die Verwendung Dekontaminationsmitteln erleichtert, die bei Raumtemperatur und Umgebungsbedingungen flüssig oder fest (vorzugsweise tablettenförmig) sind. Durch ein kondensatfreies Verdampfen wird gleichzeitig sichergestellt, dass die Dekontaminationsmittel den Innenraum der Kammer mit der vorgesehenen Konzentration vollständig ausfüllen und somit alle zugänglichen Stellen eines zu dekontaminierenden Gegenstandes zuverlässig erreichen.

Besonders bevorzugt ist es, wenn der Druckmesser dazu ausgebildet ist, eine Druckdifferenz von zumindest 1 Pa zu erfassen.

Ein solcher Druckmesser ermöglicht eine besonders genaue Ermittlung der Dekontaminationsmittelkonzentration, da schon geringe Druckunterschiede genau erfasst werden können. Die genaue Bestimmung der Konzentration des Dekontaminationsmittels in der Kammer erlaubt eine sparsame Zuführung des Dekontaminationsmittels, was zur Folge hat, dass der hohe Unterdruck auch während des Dekontaminationsprozesses weitgehend aufrechterhalten werden kann. Damit kann der gesamte Dekontaminationsprozess wirkungsvoller und zuverlässiger durchgeführt werden.

Verfahrenstechnisch ist es außerdem bevorzugt, wenn das Dekontaminationsmittel einen Sättigungsdruck definiert und der Innendruck in dem Innenraum dauerhaft unterhalb des Sättigungsdrucks gehalten wird.

In diesem Fall entspricht die über den Druckanstieg bestimmte Dekontaminationsmittelkonzentration der tatsächlich wirksamen Konzentration in der Kammer sehr genau. Die Einhaltung der Prozessparameter kann dementsprechend noch genauer und zuverlässiger gewährleistet werden.

In einer weiteren Ausgestaltung ist die zweite Anordnung so ausgebildet, dass sie den Innenraum mit Hilfe von Unterdruck mit dem gasförmigen Dekontaminationsmittel befüllt.

Alternativ hierzu ist es grundsätzlich auch möglich, das gasförmige Dekontaminationsmittel über eine Pumpe oder dgl. in den Innenraum der Kammer hineinzublasen. Die bevorzugte Ausgestaltung ist demgegenüber einfacher, da eine Pumpe zum Erzeugen eines Unterdrucks in der Kammer ohnehin vorhanden ist. Darüber hinaus ist die bevorzugte Ausgestaltung auf Grund der reduzierten Teilezahl auch leichter und sie kann kleinbauender realisiert werden, was vor allem die Mobilität in vorteilhafter Weise begünstigt.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

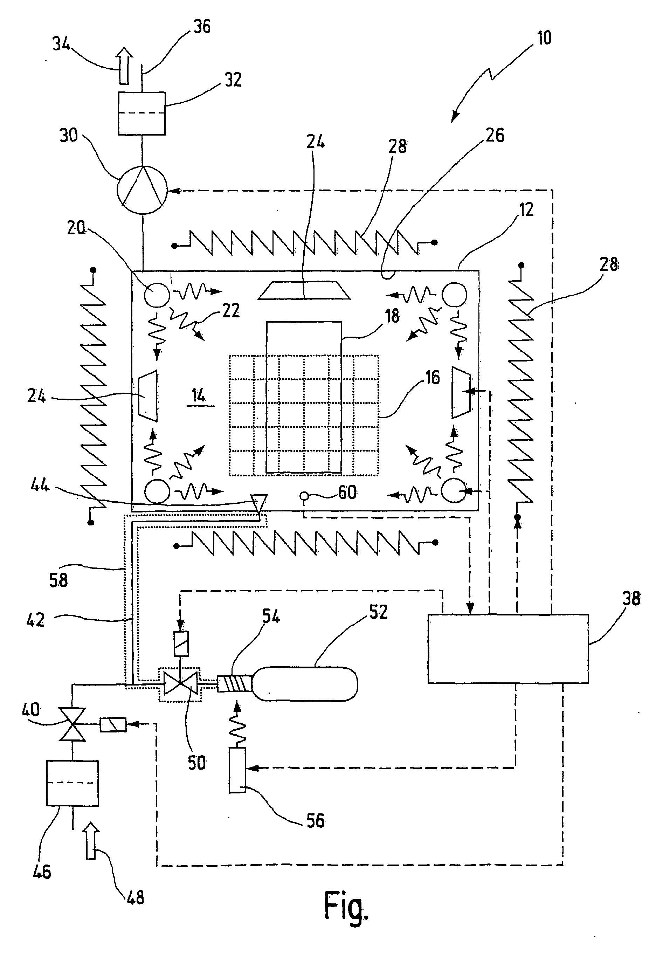

In der einzigen Figur ist ein Ausführungsbeispiel der neuen Vorrichtung schematisch dargestellt und in seiner Gesamtheit mit der Bezugsziffer 10 bezeichnet.

Die Vorrichtung 10 beinhaltet eine Vakuumkammer 12 mit einem Innenraum 14. In dem Innenraum 14 ist hier eine Gitterbox 16 dargestellt, in der sich ein zu dekontaminierender Gegenstand 18 befindet. Die Gitterbox 16 vereinfacht die Beschickung der Vakuumkammer 12, sie ist jedoch für die Realisierung der erfindungsgemäßen Vorrichtung nicht zwingend erforderlich.

In dem Innenraum 14 sind Infrarotstrahler 20 angeordnet, von denen jeder (bei Bedarf) Infrarotstrahlung 22 zum Beheizen des Gegenstandes 14 erzeugt. Beispielhaft sind hier Infrarotstrahler 20 an allen vier Ecken der Vakuumkammer 12 dargestellt. Damit ist der zu dekontaminierende Gegenstand gewissermaßen von den Infrarotstrahlern 20 umgeben, was eine besonders gleichmäßige und wirkungsvolle Aufheizung ermöglicht. Alternativ hierzu kann in anderen Ausführungsbeispielen der Erfindung vorgesehen sein, dass Infrarotstrahler beidseitig oder nur oberhalb von dem zu dekontaminierenden Gegenstand 18 angeordnet sind.

Bei der Bezugsziffer 24 sind in diesem Ausführungsbeispiel drei UV-Strahler dargestellt, mit deren Hilfe der zu dekontaminierende Gegenstand beim Entseuchen mit UV-Strahlung bestrahlt werden kann.

Entlang der Innenwände 26 der Vakuumkammer 12 sind hier weitere Heizmittel 28 angeordnet. Bevorzugt wird jede Innenwand der Vakuumkammer 12 beheizt (allseitige Beheizung), wodurch eine Kondensatbildung im Innenraum 14 der Kammer 12 besonders wirkungsvoll verhindert wird. Die Heizungen 28 sind bevorzugt als elektrische Heizungen ausgebildet. Alternativ hierzu kann die Beheizung der Innenwände 26 beispielsweise auch mit Heißluft und/oder Heißgas erfolgen, die von einem Brenner (bevorzugt ein Diesel- oder Vielstoffbrenner) erzeugt werden. Zu diesem Zweck können die Innenwänden mit geeigneten Kanälen (hier nicht dargestellt) zum Durchschleusen der Heißluft versehen sein.

Bei der Bezugsziffer 30 ist eine Vakuumpumpe dargestellt, mit deren Hilfe der Innenraum 14 der Kammer 10 evakuiert werden kann. Die Vakuumpumpe ist bevorzugt eine Drehschieberpumpe. In dem bevorzugten Ausführungsbeispiel der Erfindung ist die Vakuumpumpe 30 so ausgebildet, dass der Innenraum der Kammer 10 auf einen Innendruck von etwa 1 Pa evakuiert werden kann. Stromabwärts ist die Vakuumpumpe 30 mit einem Filter verbunden. In einem besonders bevorzugten Ausführungsbeispiel handelt es sich hier um einen sog. HEPA-Filter (High Efficiency Particle Absorber), der in der Lage ist, auch kleinste Partikel auszufiltern. Außerdem ist es bevorzugt, dass der Filter 32 mit Aktivkohle (hier nicht dargestellt) bestückt ist. Der Filter 32 ist damit in der Lage, die Abluft 34 am Auslass 36 von Schadstoffen zu befreien, die beispielsweise beim Verdampfen von chemischen Substanzen in der Vakuumkammer 10 freigesetzt wurden. Darüber hinaus ist der Filter 32 bevorzugt auch dazu ausgebildet, die Abluft 34 von toxischen oder anderweitig gefährlichen Dekontaminationsmitteln zu befreien, die zum Entseuchen eines Gegenstandes 18 verwendet wurden.

Mit der Bezugsziffer 38 ist eine Steuereinheit bezeichnet, mit deren Hilfe die Prozessparameter in definierter Weise gesteuert werden. Hierzu ist die Steuereinheit 38 insbesondere mit den Infrarotstrahlern 20, den UV-Strahlern 24, der Mantelheizung 28 und der Vakuumpumpe 30 verbunden. Mit anderen Worten werden die zuletzt genannten Elemente über die Steuereinheit 38 gezielt angesteuert. Die Steuereinheit ist dazu ausgebildet, den Dekontaminationsprozess unter Verwendung der Temperatur und des Innendrucks in der Kammer in einer definierten Weise und mit definierten Prozessparametern durchzuführen.

Bei der Bezugsziffer 40 ist ein Belüftungsventil dargestellt, dessen Öffnungs- und Schließzustand über die Steuereinheit 38 eingestellt wird. Das Belüftungsventil 40 ist über eine Zuleitung 42 mit einem Einlass 44 verbunden, der in dem Innenraum 14 der Kammer 10 mündet. Stromaufwärts von dem Belüftungsventil 40 ist ein zweiter Filter 46 angeordnet, der einströmende Frischluft 48 von Verunreinigungen befreit. Bevorzugt handelt es sich hier ebenfalls um einen HEPA-Filter mit Aktivkohle.

Bei der Bezugsziffer 50 ist ein zweites Ventil dargestellt, dessen Öffnungs- und Schließzustand über die Steuereinheit 38 eingestellt wird. Das Ventil 50 dient zum Befüllen der Kammer 12 mit einem Dekontaminationsmittel, beispielsweise Wasserstoffperoxid, Formalin, Peressigsäure oder Ethylenoxid. Die zuerst genannten Dekontaminationsmittel sind dabei bevorzugt, weil sie eine geringere Toxizität als Ethylenoxid aufweisen.

Bei der Bezugsziffer 52 ist ein Vorratsbehälter angedeutet, der zur Bereitstellung der chemischen Dekontaminationsmittel dient. In dem bevorzugten Ausführungsbeispiel beinhaltet die Vorrichtung 10 ferner einen Verdampfer 54, mit dessen Hilfe ein flüssiges Dekontaminationsmittel, beispielsweise Peressigsäure oder Formalinlösung, aus dem Vorratsbehälter 52 kondensatfrei verdampft werden kann. Der Verdampfer 54 beinhaltet dazu eine Heizung 56, die ebenfalls von der Steuereinheit 38 angesteuert wird.

Das Begasungsventil 50 ist hier ausgangsseitig an die Zuleitung 42 angeschlossen. Alternativ hierzu könnte die Frischluftzufuhr und die Zufuhr von Dekontaminationsmittel auch über vollständig getrennte Zuleitungen in die Vakuumkammer erfolgen. Die vorliegende Realisierung besitzt demgegenüber den Vorteil, dass die Zuleitung für das Dekontaminationsmittel beim Spülen der Vakuumkammer fast vollständig mitgespült wird.

In dem bevorzugten Ausführungsbeispiel ist der gesamte Zuleitungspfad zum Befüllen der Vakuumkammer 10 mit dem Dekontaminationsmittel beheizbar. Eine entsprechende Mantelheizung ist in der Figur bei der Bezugsziffer 58 schematisch dargestellt. Die Mantelheizung 58 kann dabei auch als Wärmeleiter realisiert sein, der die Abwärme von der Heizung 56 zum Beheizen der Zuleitung 42 verwendet. Bevorzugt ist es, wenn der Zuleitungspfad für das Dekontaminationsmittel vollständig beheizbar ist, so dass eine Kondensatbildung eines verdampften Dekontaminationsmittels durchgängig ausgeschlossen ist.

Bei der Bezugsziffer 60 ist ein Drucksensor dargestellt, mit dessen Hilfe die Steuereinheit 38 den Innendruck im Innenraum 14 der Kammer 12 bestimmen kann. Der Drucksensor 60 ermöglicht es, den Innendruck mit Hilfe der Vakuumpumpe 30 auf einen gewünschten Wert einzuregeln. Darüber hinaus dient der Drucksensor 60 in einem besonders bevorzugten Ausführungsbeispiel der Erfindung dazu, die Konzentration an Dekontaminationsmittel in dem Innenraum 14 der Kammer 12 einzustellen.

Zum Entgiften eines kontaminierten Gegenstandes 18 arbeitet die Vorrichtung 10 vorzugsweise wie folgt: Der Gegenstand 18 wird in den Innenraum 14 der Kammer 12 hineingelegt und die Kammer 12 wird druckdicht verschlossen. Anschließend wird mit Hilfe der Steuereinheit 38 und der Vakuumpumpe 30 der Innenraum 14 der Kammer 12 evakuiert, und zwar vorzugsweise auf einen Innendruck von etwa 1 Pa (Vakuum). Gleichzeitig mit dem Erzeugen des Vakuums oder im Anschluss daran wird der Innenraum 14 der Kammer 12 beheizt, und zwar vorzugsweise unter Verwendung der Mantelheizung 28 und der Infrarotstrahler 22. Der Innenraum 14 wird auf etwa 70°C aufgeheizt, was über einen hier nicht dargestellten Temperatursensor erfasst wird.

Infolge des Unterdrucks (Vakuums) im Innenraum 14 der Kammer 12 verdampfen vorhandene chemische Kontaminationen trotz der vergleichsweise niedrigen Temperatur. Der Innenraum 14 wird nach einer gewissen Einwirkzeit belüftet, d.h. es wird Frischluft 48 über das Belüftungsventil 40 in den Innenraum 14 eingeleitet. Anschließend oder gleichzeitig wird der Innenraum 14 evakuiert, wodurch die verdampften Kontaminationen abgesaugt werden. Die Abluft wird mit Hilfe des Filters 32 gereinigt. Vorzugsweise wird der ganze Vorgang mehrfach wiederholt, um eine optimale Entgiftung des Gegenstandes 18 zu gewährleisten.

Im Gegensatz zu den bekannten Entgiftungsverfahren wird hier also zur Dekontamination kein Heißdampf und auch kein Rauchgas eingesetzt. Die Entgiftung basiert lediglich auf der Verdampfung der chemischen Substanzen bei relativ niedrigen Temperaturen infolge des Unterdrucks bzw. Vakuums.

Für die Entseuchung eines Gegenstandes wird gleichzeitig mit oder nach dem Evakuieren des Innenraums 14 ein chemisches Dekontaminationsmittel über das Begasungsventil 50 in den Innenraum 14 eingeleitet. Ein chemisches Dekontaminationsmittel in diesem Sinne sind alle Substanzen, die biologische Kontaminationen aufgrund einer nicht ausschließlich thermisch bedingten Wechselwirkung deaktivieren. In der bevorzugten Variante des Verfahrens, die auch der hier dargestellten Vorrichtung entspricht, erfolgt das Einleiten des Dekontaminationsmittels lediglich mit Hilfe des Unterdrucks in der Kammer 12. Mit anderen Worten wird das chemische Dekontaminationsmittel aufgrund des Unterdrucks in die Kammer 12 hineingesaugt.

Ergänzend zur Anwendung des chemischen Dekontaminationsmittels kann der zu dekontaminierende Gegenstand 18 auch mit den UV-Strahlern 24 bestrahlt werden, um freie Radikale zu erzeugen, die mit den zu dekontaminierenden Substanzen wechselwirken.

In bevorzugten Ausführungsbeispielen wird als chemisches Dekontaminationsmittel Wasserstoffperoxid, Formalinlösung oder Peressigsäure verwendet. Diese Mittel werden vor Einleiten in den Innenraum 14 der Kammer 12 kondensatfrei verdampft. Die Konzentration des Dekontaminationsmittels in der Kammer 12 lässt sich bei der bevorzugten Vorrichtung mit Hilfe des Drucksensors 60 bestimmen und über die Steuereinheit 38 in der gewünschten Weise einstellen. Dazu ist es lediglich erforderlich, die Konzentration des Dekontaminationsmittels in der wässrigen Lösung im Vorratsbehälter 52 zu kennen. Des Weiteren wird der Innendruck im Innenraum 14 der Kammer 12 vor Einleiten des Dekontaminationsmittels und die Temperatur in der Kammer 12 benötigt. Da sich der Innendruck in der Kammer beim Einleiten des gasförmigen Dekontaminationsmittels erhöht, kann aus der Druckdifferenz die aktuelle Konzentration des Dekontaminationsmittels in der Kammer 12 bestimmt werden. Dabei wird das ideale Gasgesetz nach Dalton angewendet. Die Konzentration ergibt sich mit:

$$\frac{n}{V} = \frac{\Delta p}{R \cdot T}$$

wobei

$n$     die Stoffmenge ist,

$V$     das Volumen in der Kammer ist,

$\Delta p$     die Druckdifferenz zwischen dem Druck in der Kammer vor und nach dem Einleiten des verdampften Dekontaminati- onsmittels ist,

$R$     die Gaskonstante ist, und

$T$     die Temperatur in der Kammer ist.

Alternativ hierzu wäre es jedoch grundsätzlich auch möglich, geeignete Gassensoren zum messtechnischen Erfassen der jeweiligen Dekontaminationsmittelkonzentration in der Kammer 14 vorzusehen. Die bevorzugte Ausgestaltung ist demgegenüber jedoch einfacher, kostengünstiger und robuster, was vor allem für den mobilen Einsatz bei militärischen Anwendungen von Vorteil ist. Sie reduziert zudem statistische Messungenauigkeiten, die bei Verwendung von Gassensoren aufgrund der geringen Molekülzahl im Innenraum auftreten können.

Da die Bestimmung der Konzentration des Dekontaminationsmittels über die Druckdifferenz vor und nach dem Zuführen des Dekontaminationsmittels andereseits ungenau wird, wenn das gasförmig zugeführte Dekontaminationsmittel auf seinem Weg in die Kammer oder in der Kammer kondensiert, ist gerade in der zuletzt genannten Variante von besonderem Vorteil, wenn die Kondensatbildung durch die allseitige Beheizung der Innenwände 26 der Kammer 12 sowie durch die Beheizung der Zuleitung 42 verhindert wird.

Bei Verwendung eines von vornherein gasförmig vorliegenden Dekontaminationsmittels, wie beispielsweise Ethylenoxid, kann auf die exakte Druckbestimmung und die Beheizung des Zuleitungspfades verzichtet werden.

Der Vorteil der neuen Vorrichtung und des neuen Verfahrens liegt vor allem in der einfachen, jedoch zuverlässigen Einstellung der atmosphärischen Bedingungen im Innenraum 14 der Kammer 12. Durch die Gewährleistung der einmal definierten Prozessparameter lässt sich mittelbar und auf einfache Weise der Erfolg der Dekontamination sicherstellen, ohne dass dazu aufwendige Messungen oder anderweitige Analysen des Schadstoffgehaltes in der Kammer und/oder an dem zu dekontaminierenden Gegenstand durchgeführt werden müssten.

**Patentansprüche**

1. Vorrichtung zum Dekontaminieren von Gegenständen (18), insbesondere zum Entgiften oder Entseuchen von temperaturempfindlichem, militärischen Kleingerät, mit

    - einer Vakuumkammer (12) mit einem Innenraum (14) zur Aufnahme eines zu dekontaminierenden Gegenstandes (18),

    - einer Vakuumpumpe (30) zum Erzeugen eines Vakuums in dem Innenraum (14),

    - einer Heizung (20, 28) zum Aufheizen des Innenraums (14), wobei die Heizung eine Mantelheizung (28) beinhaltet, die dazu ausgebildet ist, Innenwände (26) der Vakuumkammer (12) zu beheizen,

    - einer ersten Anordnung (40, 42, 44, 46) zum Belüften des Innenraums (14),

    - einer zweiten Anordnung (42, 44, 50, 52, 54, 56) zum Befüllten des Innenraums (14) mit einem gasförmigen chemischen Dekontaminationsmittel, wobei die zweite Anordnung einen Vorratsbehälter (52) für chemische Dekontaminationsmittel beinhaltet, der so über eine Zuleitung (42) mit dem Innenraum (14) der Vakuumkammer (12) verbunden ist,

    - einem Druckmesser (60) zum Bestimmen eines Innendrucks in dem Innenraum (14), und

    - einer Steuereinheit (38), die dazu ausgebildet ist, die zweite Anordnung in Abhängigkeit von dem Innendruck anzusteuern,

    **dadurch gekennzeichnet, dass** die Vakuumpumpe (30) dazu ausgebildet ist, den Innenraum auf einen Innendruck von weniger als 10 Pa, bevorzugt auf einen Innendruck von etwa 1 Pa zu evakuieren, dass die Heizung ferner zumindest einen Wärmestrahler (20) beinhaltet, der in dem Innenraum (14) angeordnet ist, und dass die Zuleitung (42) vollständig beheizbar ist, um ein Kondensieren eines verdampften gasförmigen Dekontaminationsmittels beim Einleiten in den Innenraum (14) zu unterbinden.

2. Vorrichtung nach Anspruch 1, **dadurch gekennzeichnet, dass** der Wärmestrahler (20) ein Infrarotstrahler ist.

3. Vorrichtung nach Anspruch 1 oder 2, **dadurch gekennzeichnet, dass** der Wärmestrahler (20) und die Mantelheizung (28) unabhängig voneinander steuerbar sind.

4. Vorrichtung nach einem der Ansprüche 1 bis 3, **dadurch gekennzeichnet, dass** die Mantelheizung (28) dazu ausgebildet ist, sämtliche Innenwände (26) der Vakuumkammer (14) zu beheizen.

5. Vorrichtung nach einem der Ansprüche 1 bis 4, **dadurch gekennzeichnet, dass** die Heizung (20, 28) so ausgebildet ist, dass sie den Innenraum (14) auf bis zu 200°C aufheizen kann.

6. Vorrichtung nach einem der Ansprüche 1 bis 5, **gekennzeichnet durch** zumindest einen UV-Strahler (24), der in dem Innenraum (14) angeordnet ist.

**7.** Vorrichtung nach einem der Ansprüche 1 bis 6, **dadurch gekennzeichnet, dass** die zweite Anordnung einen Verdampfer (54, 56) zum kondensatfreien Verdampfen eines Dekontaminationsmittels beinhaltet.

**8.** Vorrichtung nach einem der Ansprüche 1 bis 7, **dadurch gekennzeichnet, dass** der Druckmesser (60) dazu ausgebildet ist, eine Druckdifferenz von zumindest 1 Pa zu erfassen.

**9.** Vorrichtung nach einem der Ansprüche 1 bis 8, **dadurch gekennzeichnet, dass** die zweite Anordnung so ausgebildet ist, dass sie den Innenraum (14) mit Hilfe von Unterdruck mit dem gasförmigen Dekontaminationsmittel befüllt.

**10.** Verfahren zum Dekontaminieren von Gegenständen, insbesondere zum Entgiften oder Entseuchen von temperaturempfindlichem, militärischen Kleingerät (18), mit den Schritten:

- Bereitstellen einer Vakuumkammer (12) mit einem Innenraum (14) und Einlegen eines zu dekontaminierenden Gegenstandes (18) in den Innenraum (14),
- Evakuieren des Innenraums mit einer Vakuumpumpe (30),
- Aufheizen des Innenraums mit einer Heizung (20, 28), die eine Mantelheizung (28) zum Beheizen der Innenwände beinhaltet, und
- Zuführen eines chemischen Dekontaminationsmittels in den Innenraum (14), um biologische Kontaminationen von den Gegenständen (18) zu entfernen, sowie
- anschließendes Belüften des Innenraums (14), **dadurch gekennzeichnet, dass**
- der Innenraum auf einen Innendruck von weniger als 100 Pa, vorzugsweise auf einen Innendruck von weniger als 10 Pa, und noch weiter bevorzugt auf einen Innendruck von etwa 1 Pa, evakuiert wird,
- der Innenraum (14) ferner mit zumindest einem Wärmestrahler (20) beheizt wird, der in dem Innenraum (14) angeordnet ist,
- ein Druckanstieg in dem Innenraum (14) beim Befüllen des Innenraums (14) mit einem gasförmigen chemischen Dekontaminationsmittel bestimmt wird, und
- das gasförmige chemische Dekontaminationsmittel in Abhängigkeit von dem Druckanstieg zugeführt wird.

**11.** Verfahren nach Anspruch 10, **dadurch gekennzeichnet, dass** unter Verwendung des Druckanstiegs eine Dekontaminationsmittelkonzentration in dem Innenraum (14) bestimmt wird und dass das Dekontaminationsmittel in Abhängigkeit von der Dekontaminationsmittelkonzentration in den Innenraum (14) zugeführt wird.

**12.** Verfahren nach einem der Ansprüche 10 oder 11, **dadurch gekennzeichnet, dass** das Dekontaminationsmittel einen Sättigungsdruck definiert und dass der Innendruck in dem Innenraum (14) dauerhaft unterhalb des Sättigungsdrucks gehalten wird.

## Claims

**1.** An apparatus for decontamination of objects (18), in particular for detoxification or disinfection of small, temperature-sensitive, military appliances, comprising

- a vacuum chamber (12) having an internal space (14) for receiving an object (18) to be decontaminated,
- a vacuum pump (30) for generating a vacuum in the internal space (14),
- a heater (20, 28) for heating the internal space (14), the heater comprising a casing heater (28) designed to heat internal walls (26) of the vacuum chamber (12),
- a first arrangement (40, 42, 44, 46) for ventilating the internal space (14),
- a second arrangement (42, 44, 50, 52, 54, 56) for filling the internal space (14) with a gaseous chemical decontamination agent, the second arrangement comprising a reservoir (52) for chemical decontamination agents, which reservoir is connected to the internal space (14) of the vacuum chamber (12) via a supply line (42),
- a pressure gauge (60) for determining an internal pressure in the internal space (14), and
- a control unit (38) designed for controlling the second arrangement as a function of the internal pressure,

**characterized in that** the vacuum pump (30) is designed to evacuate the internal space to an internal pressure of less than 10 Pa, preferably to an internal pressure of about 1 Pa, the heater further comprises at least one heat radiator (20) arranged in the internal space (14), and the supply line (42) can be completely heated in order to avoid condensation of an evaporated gaseous decontamination agent at the supply into the internal space (14).

**2.** The apparatus of claim 1, **characterized in that** heat radiator (20) is an infrared radiator.

**3.** The apparatus of claim 1 or 2, **characterized in that** the heat radiator (20) and the casing heater (28) are adapted to be controlled independently of one another.

**4.** The apparatus of one of claims 1 to 3, **characterized in that** the casing heater (28) is designed to heat all of the internal walls (26) of the vacuum chamber (14).

**5.** The apparatus of one of claims 1 to 4, **characterized in that** the heater (20, 28) is designed to heat the internal space (14) up to 200°C.

**6.** The apparatus of one of claims 1 to 5, **characterized by** at least one UV radiator (24) arranged in the internal space (14).

**7.** The apparatus of one of claims 1 to 6, **characterized in that** the second arrangement comprises a vaporizer (54, 56) for condensation-free vaporization of a decontamination agent.

**8.** The apparatus of one of claims 1 to 7, **characterized in that** the pressure gauge (60) is designed to detect a pressure difference of at least 1 Pa.

**9.** The apparatus of one of claims 1 to 8, **characterized in that** the second arrangement is designed such that it fills the internal space (14) with the gaseous decontamination agent by means of vacuum pressure.

**10.** A method for decontamination of objects, in particular for detoxification or disinfection of small, temperature-sensitive military appliances (18), comprising the following steps:

- providing a vacuum chamber (12) having an internal space (14), and inserting an object (18) to be decontaminated into the internal space (14),
- evacuating the internal space by means of a vacuum pump (30),
- heating the internal space by means of a heater (20, 28) which comprises a casing heater (28) for heating internal walls, and
- supplying a chemical decontamination agent into the internal space (14) for removing biological contamination from the object (18), and
- subsequent ventilation of the internal space (14),
**characterized in that**
- the internal space is evacuated to an internal pressure of less than 100 Pa, preferably to an internal pressure of less than 10 Pa, and even more preferably to an internal pressure of about 1 Pa,
- the internal space (14) is further heated by means of at least one heat radiator which is arranged in the internal space (14),
- a pressure rise in the internal space is determined while the internal space (14) is being filled with a gaseous chemical decontamination agent, and
- the gaseous chemical decontamination agent is supplied in dependence of the pressure rise.

**11.** The method of claim 10, **characterized in that** a concentration of the decontamination agent in the internal space (14) is determined using the pressure rise, and **in that** the decontamination agent is supplied to the internal space (14) as a function of the decontamination agent concentration.

**12.** The method of one of claims 10 or 11, **characterized in that** the decontamination agent defines a saturation pressure, and **in that** the internal pressure in the internal space (14) is constantly kept below the saturation pressure.

**Revendications**

**1.** Dispositif pour décontaminer des objets (18), en particulier pour désinfecter ou décontaminer du petit matériel militaire et sensible à la température comprenant :

- une chambre à vide (12) avec un espace intérieur (14) pour le logement d'un objet (18) à décontaminer,
- une pompe à vide (30) pour générer un vide dans l'espace intérieur (14),
- un chauffage (20, 28) pour le réchauffement de l'espace intérieur (14), le chauffage contenant un chauffage d'enveloppe (28), qui est conçu pour chauffer des parois intérieures (26) de la chambre à vide (12),
- un premier agencement (40, 42, 44, 46) pour l'aération de l'espace intérieur (14),
- un second agencement (42, 44, 50, 52, 54, 56) pour le remplissage de l'espace intérieur (14) avec un agent de décontamination chimique gazeux, le second agencement contenant un réservoir de stockage (52) pour des agents de décontamination chimiques, qui est relié ainsi au moyen d'une arrivée (42) à l'espace intérieur (14) de la chambre à vide (12),
- un mesureur de pression (60) pour déterminer une pression intérieure dans l'espace intérieur (14), et
- une unité de commande (38), qui est conçue pour activer le second agencement en fonction de la pression intérieure,

**caractérisé en ce que** la pompe à vide (30) est conçue pour faire le vide dans l'espace intérieur jusqu'à une pression intérieure de moins de 10 Pa, de préférence jusqu'à une pression intérieure d'environ 1 Pa, **en ce que** le chauffage contient également au moins un radiateur thermique (20), qui est disposé dans l'espace intérieur (14), et **en ce que** l'arrivée (42) peut être chauffée complètement pour supprimer une condensation d'un agent de décontamination gazeux évaporé lors de l'introduction dans l'espace intérieur (14).

2. Dispositif selon la revendication 1, **caractérisé en ce que** le radiateur thermique (20) est un radiateur infrarouge.

3. Dispositif selon la revendication 1 ou 2, **caractérisé en ce que** le radiateur thermique (20) et le chauffage d'enveloppe (28) peuvent être commandés indépendamment l'un de l'autre.

4. Dispositif selon l'une quelconque des revendications 1 à 3, **caractérisé en ce que** le chauffage d'enveloppe (28) est conçu pour chauffer toutes les parois intérieures (26) de la chambre à vide (14).

5. Dispositif selon l'une quelconque des revendications 1 à 4, **caractérisé en ce que** le chauffage (20, 28) est conçu de telle sorte qu'il puisse chauffer l'espace intérieur (14) jusqu'à 200°C.

6. Dispositif selon l'une quelconque des revendications 1 à 5, **caractérisé par** au moins un radiateur UV (24), qui est disposé dans l'espace intérieur (14).

7. Dispositif selon l'une quelconque des revendications 1 à 6, **caractérisé en ce que** le second agencement contient un évaporateur (54, 56) pour l'évaporation sans condensat d'un agent de décontamination.

8. Dispositif selon l'une quelconque des revendications 1 à 7, **caractérisé en ce que** le mesureur de pression (60) est conçu pour enregistrer une différence de pression d'au moins 1 Pa.

9. Dispositif selon l'une quelconque des revendications 1 à 8, **caractérisé en ce que** le second agencement est conçu de telle sorte qu'il remplisse l'espace intérieur (14) à l'aide d'une dépression avec l'agent de décontamination gazeux.

10. Procédé pour décontaminer des objets, en particulier pour désinfecter ou décontaminer du petit matériel (18) militaire et sensible à la température comprenant les étapes suivantes :

- mise à disposition d'une chambre à vide (12) avec un espace intérieur (14) et introduction d'un objet (18) à décontaminer dans l'espace intérieur (14),
- production de vide dans l'espace intérieur avec une pompe à vide (30),
- chauffage de l'espace intérieur avec un chauffage (20, 28) qui contient un chauffage d'enveloppe (28) pour le chauffage des parois intérieures, et
- arrivée d'un agent de décontamination chimique dans l'espace intérieur (14) pour enlever des contaminations biologiques des objets (18), et
- aération consécutive de l'espace intérieur (14), **caractérisé en ce que**,
- l est fait le vide dans l'espace intérieur jusqu'à une pression intérieure de moins de 100 Pa, de préférence jusqu'à une pression intérieure de moins de 10 Pa, plus préférentiellement jusqu'à une pression intérieure d'environ 1 Pa.
- space intérieur (14) est chauffé également avec au moins un radiateur thermique (20), qui est disposé dans l'espace intérieur (14),
- une augmentation de pression dans l'espace intérieur (14) est déterminée lors du remplissage de l'espace intérieur (14) avec un agent de décontamination chimique gazeux, et
- l'agent de décontamination chimique gazeux est amené en fonction de l'augmentation de pression.

11. Procédé selon la revendication 10, **caractérisé en ce qu'**une concentration d'agent de décontamination dans l'espace intérieur (14) est déterminée en utilisant l'augmentation de pression et **en ce que** l'agent de décontamination est amené dans l'espace intérieur (14) en fonction de la concentration d'agent de décontamination.

12. Procédé selon la revendication 10 ou 11, **caractérisé en ce que** l'agent de décontamination définit une pression de saturation et **en ce que** la pression intérieure dans l'espace intérieur (14) est maintenue de façon durable au-dessous de la pression de saturation.

Fig.

**IN DER BESCHREIBUNG AUFGEFÜHRTE DOKUMENTE**

**In der Beschreibung aufgeführte Patentdokumente**

- US 5122344 A
- DE 3429346 A1
- DE 3625847 A1
- DE 3413743 A1
- DE 3835857 A1
- DE 12003
- DE 2921915 A1
- DE 69821825 T2
- EP 0109352 A
- DE 3842285 A1